(19) **Europäisches Patentamt / European Patent Office / Office européen des brevets**

(11) **EP 1 838 246 B1**

(12) # EUROPEAN PATENT SPECIFICATION

(45) Date of publication and mention
of the grant of the patent:
**17.02.2010 Bulletin 2010/07**

(21) Application number: **05852541.1**

(22) Date of filing: **01.12.2005**

(51) Int Cl.:
***A61F 2/16*** (2006.01)

(86) International application number:
**PCT/US2005/043324**

(87) International publication number:
**WO 2006/060480 (08.06.2006 Gazette 2006/23)**

(54) **APODIZED ASPHERIC DIFFRACTIVE LENSES**

APODISIERTE ASPHÄRISCHE DIFFRAKTIVE LINSEN

LENTILLES DIFFRACTIVES ASPHERIQUES APODISEES

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.12.2004 US 770**

(43) Date of publication of application:
**03.10.2007 Bulletin 2007/40**

(73) Proprietor: **Alcon, Inc.
6331 Hünenberg (CH)**

(72) Inventor: **SIMPSON, Michael, J.
Arlington, Texas 76012 (US)**

(74) Representative: **Hanna, Peter William Derek
Hanna Moore & Curley
13 Lower Lad Lane
Dublin 2 (IE)**

(56) References cited:
**WO-A-01/82791       WO-A-2005/098518
US-A- 5 824 074       US-A1- 2004 156 014**

**Description**

## TECHNICAL FIELD OF THE INVENTION

The present invention relates generally to multifocal diffractive ophthalmic lenses and, more particularly, to apodized diffractive intraocular lenses that can provide enhanced image contrast.

Periodic diffractive structures can diffract light simultaneously into several directions, also typically known as diffraction orders. In multifocal intraocular lenses, two diffraction orders are utilized to provide a patient with two optical powers, one for distance vision and the other for near vision. Such diffractive intraocular lenses are typically designed to have an "add" power that provides a separation between the far focus and the near focus. In this manner, a diffractive intraocular lens can provide a patient in whose eye the lens is implanted with vision over a range of object distances. For example, a diffractive IOL can replace a patient's natural lens to provide the patient not only with a requisite optical power but also with some level of pseudoaccommodation. In another application, a diffractive IOL or other ophthalmic lens can provide the eye of a patient who suffers from presbyopia - a loss of accommodation of the natural lens - with pseudoaccommodative ability.

US 2004/156014-A1 (Piers et al) discloses an apodized multifocal diffractive lens which is equi-biconvex, having similar base profiles on an anterior and a posterior surface thereof.

US-A- 5,824,074 (Koch et al) describes improving the optical quality of a dual intraocular lens where one lens may be rotated relative to the other about an optical axis, each of which displays a compensation section, which may be toric, for compensating astigmatism, when the lenses are rotated relative to one another.

Conventional multifocal diffractive lenses, however, are not designed so as to control or modify aberrations of the natural eye such that the combined lens and the patient eye would provide enhanced image contrast. Moreover, the design of apodized diffractive lenses for providing better image contrast can present difficulties in that such lenses exhibit a varying diffractive effect at different radial locations across the lens.

## BRIEF SUMMARY OF THE INVENTION

The present invention generally provides multifocal ophthalmic lenses, such as intraocular and contact lenses, that employ aspherical surface profiles to enhance image contrast, particularly at a far focus of the lens. In many embodiments, the invention provides pseudoaccommodative lenses having at least one aspherical surface for enhancing image contrast.

In one aspect, the present invention provides a diffractive lens, such as a pseudoaccommodative intraocular lens (IOL), that includes an optic having an aspherical base curve, and a plurality of annular diffractive zones superimposed on a portion of the base curve so as to generate a far focus and a near focus. The aspherical base curve enhances image contrast at the far focus of the optic relative to that obtained by a substantially identical IOL in which the respective base curve is spherical.

The image enhancement provided by the aspherical base curve can be characterized by a modulation transfer function (MTF) exhibited by the combined IOL and a patient's eye in which the IOL is implanted. For example, such an MTF at the far focus can be greater than about 0.2 (e.g., in a range of about 0.2 to about 0.5) when calculated in a model eye at a spatial frequency of about 50 line pairs per millimeter (lp/mm) or greater than about 0.1 (e.g., in a range of about 0.1 to about 0.4) at a spatial frequency of about 100 lp/mm, a wavelength of about 550 nm and a pupil size of about 4 mm to about 5 mm. More preferably, the MTF can be greater than 0.3, or 0.4. For example, the MTF can be in a range of about 0.2 to about 0.5. For example, the calculated MTF can be greater than about 0.2 at a spatial frequency of about 50 lp/mm, a wavelength of about 550 nm, and a pupil size of about 4.5 mm.

In another aspect, the aspherical profile is selected to strike a balance between enhancing image contrast and providing a useful depth of field. Rather than correcting all aberrations, the lens can be configured such that the combined IOL and a patient's eye in which the IOL is implanted can exhibit a useful depth of field, particularly at the far focus. The terms "depth of field" and "depth of focus," which are herein used interchangeably, are well known in the context of a lens and readily understood by those skilled in the art. To the extent that a quantitative measure may be required, the term "depth of field" or "depth of focus" as used herein, can be determined by an amount of defocus associated with the optical system at which a through-focus modulation transfer function (MTF of the system calculated or measured with an aperture, e.g., a pupil size, of about 4 mm to about 5 mm (e.g., a pupil size of about 4.5 mm) and monochromatic green light, e.g., light having a wavelength of about 550 nm, exhibits a contrast of at least about 0.3 at a spatial frequency of about 50 lp/mm or a contrast of about 0.2 at a spatial frequency of about 100 lp/mm. It should be understood the depth of field at the far focus refers to a defocus distance less than the separation between the far focus and the near focus, i.e., it refers to a depth of field when the patient is viewing a far object.

In a related aspect, the diffractive zones can be disposed within a portion of a lens surface, herein referred to as the apodization zone, surrounded by a peripheral portion of the surface that is substantially devoid of diffractive structures. The diffractive zones can be separated from one another by a plurality of steps located at zone boundaries that have substantially uniform heights. Alternatively, the step heights can be non-uniform. For example, the step heights can progressively decrease as a function of increasing distance from the lens's optical axis.

In some embodiments, the lens includes an anterior surface having the aspherical profile and a posterior surface that is spherical. Alternatively, the posterior surface can be aspherical and the anterior surface spherical. In other embodiments, both the anterior surface and the posterior surface can be aspherical, that is, a total desired degree of asphericity can be divided between the anterior and the posterior surfaces.

In a related aspect, the asphericity of one or more surfaces of the IOL can be characterized by the following relation:

$$z = \frac{cR^2}{1 + \sqrt{1 - (1 + cc)c^2 R^2}} + adR^4 + aeR^6 + \text{higher order terms} \ ,$$

wherein

$z$ denotes a sag of the surface parallel to an axis (z), e.g., the optical axis, perpendicular to the surface,

$c$ denotes a curvature at the vertex of the surface,

$cc$ denotes a conic coefficient,

$R$ denotes a radial position on the surface,

$ad$ denotes a fourth order deformation coefficient, and

$ae$ denotes a sixth order deformation coefficient.

Distances are given herein in units of millimeters. For example, the curvature constant is given in units of inverse millimeter, while $ad$ is given in units of $\dfrac{1}{(\text{mm})^3}$ and $ae$ is given in units of $\dfrac{1}{(\text{mm})^5}$.

The parameters in the above relation can be selected based, e.g., on the desired optical power of the lens, the material from which the lens is formed, and the degree of image enhancement expected from the asphericity of the profile. For example, in some embodiments in which the lens optic is formed as a biconvex lens of an acrylic polymeric material of average power (e.g., a power of 21 Diopters), the conic constant ($cc$) of the anterior surface can be in a range of about 0 (zero) to about -50 (minus fifty), or in a range of about -10 (minus 10) to about -30 (minus 30), or a range of about -15 (minus 15) to about - 25 (minus 25), and the deformation constants ($ad$) and ($ae$) can be, respectively, in a range of about 0 to about -1x10$^{-3}$ (minus 0.001) and in a range of about 0 to about -1x10$^{-4}$ (minus 0.0001).

In another aspect, the present invention provides a pseudoaccommodative apodized diffractive IOL that includes an optic having an anterior surface and a posterior surface, wherein at least one of the surfaces includes an aspherical base profile and a plurality of diffractive zones superimposed on a portion of the base profile such that each zone is disposed at a selected radius from an optical axis of the optic and is separated from an adjacent zone by a step. This lens surface can further include a peripheral region surrounding the diffractive zones. The diffractive zones generate a far focus and a near focus and the aspherical profile enhances the image contrast at the far focus relative to that obtained by a substantially identical lens having a spherical profile.

In other aspects, the present invention provides a pseudoaccommodative, diffractive IOL that includes an optic formed of a biocompatible polymeric material and having a posterior surface and an anterior surface, where the optic provides a near focus and a far focus. At least one of the anterior or the posterior surfaces can be characterized by a base curve and a plurality of diffractive zones disposed as annular concentric diffractive elements about an optical axis, where each has a height relative to the base curve that progressively decreases as a distance of the diffractive element from the optical axis increases. The base curve can exhibit an aspherical profile for enhancing the image contrast at the far focus for pupil diameters in a range of about 4 to about 5 millimeters relative to a substantially identical IOL in which the base curve is spherical.

In other aspects, the invention provides an apodized diffractive ophthalmic lens that includes an optic having an anterior surface and a posterior surface, at least one of which has an aspherical base profile and a plurality of annular diffractive zones disposed on the base profile for generating a near focus and a far focus. The aspherical profile enhances an image contrast at the far focus relative to that obtained by a substantially identical lens in which a respective base profile is spherical. The ophthalmic lens can be, without limitation, an intraocular lens or a contact lens.

In another aspect, the invention provides methods for calculating optical properties of apodized diffractive lenses, and particularly apodized diffractive lenes that have at least one aspherical surface. Apodized diffractive lenses incorporate aspects of both diffraction and apodization. Hence, both of these aspects need to be included in the lens design. In particular, apodized diffractive lenses exhibit a variation of the diffractive effect at different radial locations across the lens, which can affect image contrast. Conventional aberrations, such as spherical aberration, caused by the shape of the cornea of the eye are normally calculated with the expectation that light transmission is constant across the lens surface. For example, every ray traced through an optical system in a standard raytrace program is given an equal weight. Such a conventional approach is, however, not suitable for apodized diffractive lenses in which optical transmission can vary in different regions of the lens. Rather, principles of physical optics need to be applied in performing optical calculations for apodized lenses. For example, as discussed in more detail below, in a method according to the invention apodization can be modeled as a reduction in the optical transmission through different regions of the lens.

In a related aspect, the invention provides a method of calculating a modulation transfer function (MTF) for an apodized diffractive lens having a plurality of annular diffractive structures disposed at selected radial distances from an optical axis of the lens by determining an apodization function that is indicative of diffraction efficiencies at a plurality of radial locations from an optical axis for directing light into a selected diffraction order of the lens. The apodization function can be integrated over a selected aperture so as to determine a fraction of light energy diffracted into the diffraction order. A preliminary MTF (e.g., calculated by assuming that the IOL lacks the diffractive structures) can be scaled in accordance with the integrated apodization function to generate the desired MTF.

A pseudoaccommodative, diffractive IOL according to the teachings of the invention can find a variety of applications. For example, it can be utilized in both pseudphakic and phakic patients. For example, such an IOL having a low base power (or a zero base power) can be employed as an anterior chamber lens in phakic patients.

Further understanding of the invention can be obtained by reference to the following detailed description in conjunction with the associated drawings, which are described briefly below.

## BRIEF DESCRIPTION OF THE SEVERAL VIEWS OF THE DRAWINGS

A more complete understanding of the present invention and the advantages thereof may be acquired by referring to the following description, taken in conjunction with the accompanying drawings in which like reference numbers indicate like features and wherein:

FIGURE 1A is a schematic front view of an apodized diffractive lens having an aspherical anterior surface according to one embodiment of the invention;

FIGURE 1B is a schematic cross-sectional view of an optic of the diffractive lens of FIGURE 1A illustrating a plurality of diffractive structures superimposed on an aspherical base profile of the anterior surface;

FIGURE 1C schematically depicts an aspherical base profile of the anterior surface of the lens of FIGURES 1A and 1B in relation to a putative spherical profile;

FIGURE 2 is a cross-sectional view of an apodized diffractive lens according to another embodiment of the invention in which the heights of a plurality of diffractive structures decrease as a function of increasing distance from the lens's optical axis;

FIGURE 2B schematically depicts an aspherical profile of a surface of the lens of FIGURE 2 in comparison with a putative spherical profile;

FIGURE 3A is a graph depicting an in-focus modulation transfer function (MTF) calculated in a model eye for an aspherical apodized diffractive lens according to one embodiment of the invention;

FIGURE 3B is a graph depicting an in-focus modulation transfer function (MTF) calculated in a model eye for an apodized diffractive lens substantially identical to the lens of FIGURE 3A but having spherical surface profiles;

FIGURE 4A presents a plurality of graphs depicting modulation transfer functions calculated in a model eye at 50 lp/mm and a pupil size of 4.5 mm for each of several exemplary aspherical apodized diffractive lenses combined with corneas exhibiting a range of asphericity, as well as a control graph exhibiting corresponding MTFs for substantially identical lenses having spherical profiles;

FIGURE 4B presents a plurality of graphs depicting modulation transfer functions calculated in a model eye at 100 lp/mm and a pupil size of 4.5 mm for each of several exemplary aspherical apodized diffractive lenses combined with corneas exhibiting a range of asphericity, as well as a control graph exhibiting corresponding MTFs for substantially identical lenses having spherical profiles;

FIGURE 5 schematically depicts diffractive structures of an IOL according to one embodiment of the invention which exhibit progressively decreasing heights as a function of increasing distance from the optical axis (the base curve is not shown);

FIGURE 6A depicts graphs corresponding to calculated fractional diffraction efficiency for the zeroth and first diffraction orders of the lens depicted schematically in FIGURE 5;

FIGURE 6B depicts graphs corresponding to light energy directed to the zeroth and first order foci of FIGURE 5 obtained by integrating the diffraction efficiency data presented in FIGURE 6A;

FIGURE 7A schematically depicts an exaggerated aspherical profile along one surface direction of a toric surface of an IOL according to one embodiment of the invention; and

FIGURE 7B schematically depicts an exaggerated aspherical profile along another surface direction of the toric surface associated with the profile shown in FIGURE 7A.

## DETAILED DESCRIPTION OF THE INVENTION

The present invention provides multifocal ophthalmic lenses that include at least one aspherical lens surface having an asphericity selected to improve image contrast relative to that provided by a substantially identical lens in which the respective surface is spherical. In the embodiments below, the teachings of the invention are illustrated primarily in connection with intraocular lenses. It should, however, be understood that these teachings apply equally to a variety of other ophthalmic lenses, such as contact lenses.

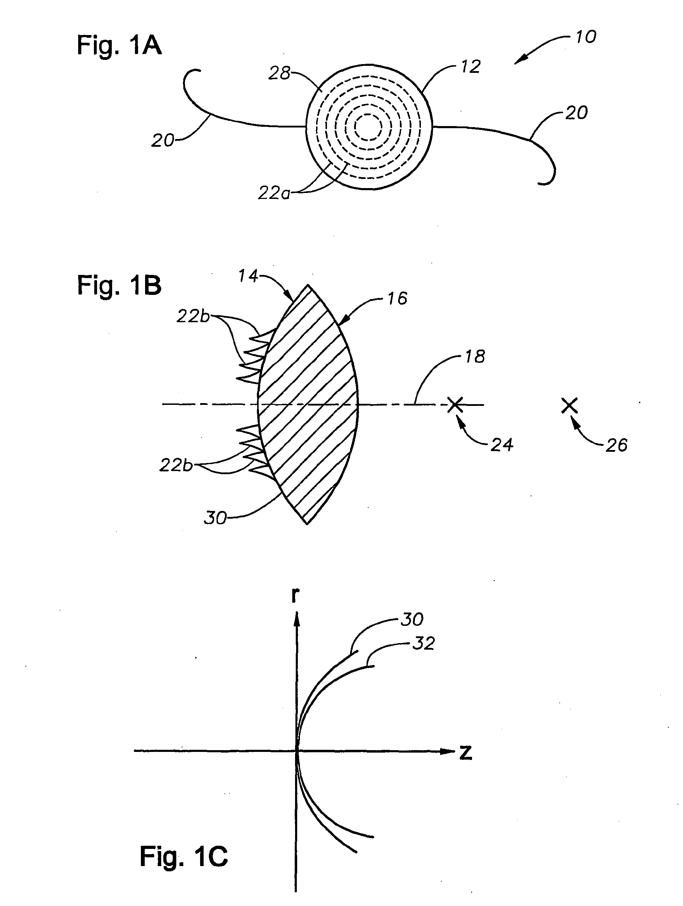

FIGURES 1A and 1B schematically illustrate a multifocal diffractive intraocular lens 10 according to one embodiment of the invention having an optic 12 that includes an anterior surface 14 and a posterior surface 16. In this embodiment, the anterior surface and the posterior surface are symmetric about an optical axis 18 of the lens, although asymmetric surfaces can also be employed. The lens further includes radially extending fixation members or haptics 20 for its placement in a patient's eye. The optic 12 can be formed of a biocompatible polymeric material, such as soft acrylic, silicone or hydrogel materials. In fact, any biocompatible - preferably soft - material that exhibits a requisite index of refraction for a particular application of the lens can be employed. Further, the fixation members 20 can be also be formed of suitable polymeric materials, such as polymethyl methacrylate, polypropylene and the like. Although the surfaces 14 and 16 are depicted as being generally convex, either surface can have a generally concave shape. Alternatively, the surfaces 14 and 16 can be selected to provide a plano-convex or a plano-concave lens. The terms "intraocular lens" and its abbreviation IOL are used interchangeably herein to describe lenses that are implanted into the interior of an eye to either replace the natural lens or to otherwise augment vision regardless of whether or not the natural lens is removed.

The anterior surface of the illustrated IOL includes a plurality of annular diffractive zones 22a providing nearly periodic microscopic structures 22b to diffract light into several directions simultaneously (the sizes of the diffractive structures are exaggerated for clarity). Although in general, the diffractive structures can be designed to divert light into more than two directions, in this exemplary embodiment, the diffractive zones cooperatively direct light primarily into two directions, one of which converges to a near focus 24 and the other to a far focus 26, as shown schematically in FIGURE 1B. Although a limited number of diffractive zones are illustrated herein, the number of the zones can generally be selected to suit a particular application. For example, the number of the diffractive zones can be in a range of about 5 to about 30. In many embodiments, the optical power associated with the far focus can be in a range of about 18 to 26 Diopters with the near focus providing an add power of about 4 Diopters. Although in this illustrative embodiment, the IOL 10 has a positive optical power, in some other embodiments, it can have a negative optical power with a positive add power separating the near focus from the far focus. The diffractive zones are confined within a portion of the surface, herein referred to as the apodization zone, and are surrounded by a peripheral portion 28 of the anterior surface that is devoid of such diffractive structures. In other words, the IOL 10 is an "apodized diffractive lens." That is, the IOL 10 exhibits a non-uniform diffraction efficiency across the anterior lens surface 14, as discussed in more detail below. Apodization can be achieved by providing diffractive structures within a region of a lens surface (referred to as the apodization zone) surrounded by a peripheral surface portion that is devoid of such diffractive structures. Hence, apodization includes both the lens region referred to as the apodization zone and the peripheral/outer lens region.

As shown schematically in FIGURE 1C, the anterior surface 14 can be characterized by a base curve 30, which depicts a profile of the surface as a function of radial distance (r) from the optical axis, on a portion of which the diffractive zones 22 are superimposed. Each diffractive zone is separated from an adjacent zone by a step whose height is related to the design wavelength of the lens in accordance with the following relation:

$$\text{step height} = \frac{\lambda}{2(n_2 - n_1)} \qquad\qquad \text{Equation (1),}$$

wherein

$\lambda$ is the design wavelength (e.g., 550 nm),

$n_2$ is the refractive index of the optic, and

$n_1$ is the refractive index of the medium surrounding the lens.

In one embodiment in which the surrounding medium is the aqueous humor having an index of refraction of 1.336, the refractive index of the optic ($n_2$) is selected to be 1.55. The uniform step height provided by the above equation is one example. Other uniform step heights can also be employed (which can change the energy balance between near and far images).

In this embodiment, the heights of the steps between different diffractive zones of the IOL 10 are substantially uniform, thereby resulting in an abrupt transition from the apodization zone to the outer portion of the lens. In other embodiments, such as those discussed in more detail below, the step heights can be non-uniform, e.g., they can progressively decrease as their distances from the optical axis increase.

The boundary of each annular zone (e.g., radius $r_i$ of the i$^{th}$ zone) relative to the optical axis can be selected in a variety of ways known to those skilled in the ophthalmic art.

With reference to FIGURE 1C, the base profile 30 of the anterior surface is aspherical with a selected degree of deviation from a putative spherical profile 32 that substantially coincides with the aspherical profile at small radial distances (i.e., at locations close to the optical axis). In this exemplary embodiment, the posterior surface has a spherical profile. In other embodiments, the posterior surface can be aspherical while the anterior surface is spherical. Alternatively, both the posterior surface and the anterior surface can be aspherical so as to provide the lens with a desired total asphericity. In this embodiment, the profile 30 of the anterior surface is generally flatter than the putative spherical profile with a deviation from the spherical profile that becomes more pronounced with increasing distance from the optical axis. As discussed in more detail below, a more pronounced asphericity within a peripheral portion of the lens can be particularly beneficial in enhancing image contrast at the far focus, as this portion is particularly efficient in directing light to the far focus. In other embodiments, the aspherical anterior surface can be steeper than the putative spherical profile.

The terms 'aspherical base curve" and "aspherical profile" are used herein interchangeably, and are well known to those skilled in the art. To the extent that any further explanation may be required, these terms are employed herein to refer to a radial profile of a surface that exhibits deviations from a spherical surface. Such deviations can be characterized, for example, as smoothly varying differences between the aspherical profile and a putative spherical profile that substantially coincides with the aspherical profile at the small radial distances from the apex of the profile. Further, the terms "substantially identical IOL" or "substantially identical lens," as used herein refer to an IOL that is formed of the same material as an aspherical IOL of the invention to which it is compared. Each surface of the "substantially identical IOL" has the same central radius (i.e., radius at the apex of the surface corresponding to the intersection of an optical axis with the surface) as that of the corresponding surface of the aspherical IOL. In addition, the "substantially identical IOL" has the same central thickness as the aspherical IOL to which it is compared. However, "substantially identical IOL" has spherical surface profiles; i.e., it lacks the asphericity exhibited by the aspherical IOL.

In many embodiments, the asphericity of the surface is selected to enhance, and in some cases maximize, the image contrast of a patient in which the IOL is implanted relative to that provided by a substantially identical IOL in which the anterior surface has the putative spherical profile 32 rather than the aspherical profile 30. For example, the aspherical profile can be designed to provide the patient with an image contrast characterized by a modulation transfer function (MTF) of at least about 0.2 at the far focus measured or calculated with monochromatic light having a wavelength of about 550 nm at a spatial frequency of 100 line pairs per millimeter (corresponding to 20/20 vision) and an aperture (e.g., pupil size) of about 4.5 mm. The MTF can be, for example, in a range of about 0.2 to about 0.5. As direct measurements of MTF in a patient's eye can be complicated, in many embodiments the image enhancement provided by an aspherical apodized diffractive IOL according to the teachings of the invention can be evaluated by calculating an MTF theoretically in a model eye exhibiting selected corneal and/or natural lens aberrations corresponding to an individual patient's eye or the eyes of a selected group of patients. The information needed to model a patient's cornea and/or natural lens can be obtained from measurements of waveform aberrations of the eye performed by employing known topographical methods.

As known to those having ordinary skill in the art, a measured or calculated modulation transfer function (MTF) associated with a lens can provide a quantitative measure of image contrast provided by that lens. In general, a contrast or modulation associated with an optical signal, e.g., a two-dimensional pattern of light intensity distribution emanated from or reflected by an object to be imaged or associated with the image of such an object, can be defined in accordance with the following relation:

$$\frac{I_{max} - I_{min}}{I_{max} + I_{min}} \qquad \text{Equation (2),}$$

wherein $I_{max}$ and $I_{min}$ indicate, respectively, a maximum or a minimum intensity associated with the signal. Such a contrast can be calculated or measured for each spatial frequency present in the optical signal. An MTF of an imaging optical system, such as the combined IOL and the cornea, can then be defined as a ratio of a contrast associated with an image of an object formed by the optical system relative to a contrast associated with the object. As is known, the MTF associated with an optical system is not only dependent on the spatial frequencies of the intensity distribution of the light illuminating the system, but it can also be affected by other factors, such as the size of an illumination aperture, as well as by the wavelength of the illuminating light.

In some embodiments, the asphericity of the anterior surface 14 is selected so as to provide a patient in which the IOL is implanted with an image contrast characterized by a modulation transfer function (MTF) that is greater than about 0.2, while maintaining a depth of field that is within an acceptable range. Both the MTF and the depth of field can be calculated in a model eye.

In some embodiments, the aspherical profile of the anterior surface 14 of the IOL 10 as a function of radial distance (R) from the optical axis 18, or that of the posterior surface or both in other embodiments, can be characterized by the following relation:

$$z = \frac{cR^2}{1 + \sqrt{1 - (1 + cc)c^2 R^2}} + adR^4 + aeR^6 + \text{ higher order terms} \qquad \text{Equation (3),}$$

wherein
$z$ denotes a sag of the surface parallel to an axis (z), e.g., the optical axis, perpendicular to the surface,
$c$ denotes a curvature at the vertex of the surface,
$cc$ denotes a conic coefficient,
$R$ denotes a radial position on the surface,
$ad$ denotes a fourth order deformation coefficient, and
$ae$ denotes a sixth order deformation coefficient.

Although in some embodiments, the conic constant cc alone is adjusted to obtain a desired deviation from sphericity, in other embodiments, in addition to the conic constant *cc*, one or both of the higher order constants *ad* and *ae* (and in particular *ae*) that more significantly affect the profile of the outer portion of the surface are adjusted to provide a selected aspherical profile for one or both surfaces of an IOL. The higher order aspherical constants (*ad* and *ae*) can be particularly useful for tailoring the profile of the peripheral portion of the lens surface, i.e., portions far from the optical axis.

The choice of the aspherical constants in the above relation for generating a desired spherical profile can depend, for example, on the aberrations of the eye in which the IOL is implanted, the material from which the IOL is fabricated, and the optical power provided by the IOL. In general, these constants are selected such that the combined IOL and the cornea, or the combined IOL, the cornea and the natural lens, provide an image contrast characterized by an MTF, e.g., an MTF calculated in a model eye, greater than about 0.2 at a spatial frequency of about 100 lp/mm, a wavelength of about 550 nm, and a pupil size of about 4.5 mm. For example, in some embodiments in which the IOL is fabricated from an acrylic polymeric material (e.g., a copolymer of acrylate and methacrylate) for implantation in an eye exhibiting a corneal asphericity characterized by a conic constant in the range of zero (associated with severe spherical aberration) to about - 0.5 (associated with a high level of aspherical flattening), the conic constant cc for the IOL in relation to the above parameters can be in a range of about 0 to about - 50 (minus fifty), or in a range of about -10 (minus 10) to about -30 (minus 30), or in a range of about -15 (minus 15) to about -25 (minus 25), while the deformation coefficients *ad* and *ae* can be, respectively, in a range of about 0 to about $\pm 1 \times 10^{-3}$ and in a range of about 0 to about $\pm 1 \times 10^{-4}$. While in some embodiments, the conic constant alone is non-zero, in other embodiments, the coefficients ad and ae are non-zero with the conic coefficient set to zero. More typically, all three aspheric coefficients *cc, ad,* and *ae*, and possibly higher order constants, are set to non-zero values to define a profile of interest. Further, the curvature coefficient (c) can be selected based on a desired optical power of the lens, the material from which the lens is formed and the curvature of the lens's other surface in a manner known in the art.

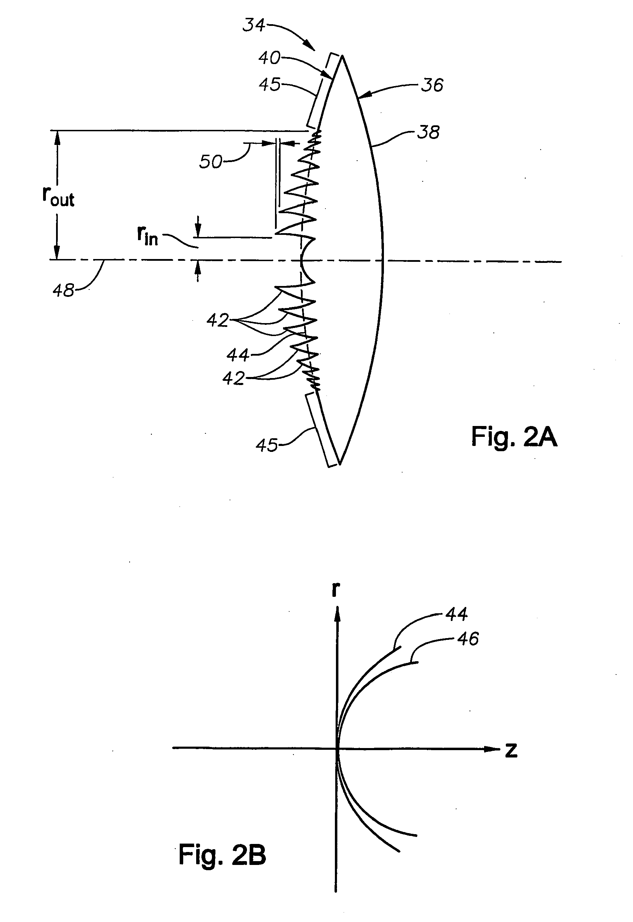

With reference to FIGURES 2A and 2B, a diffractive intraocular lens 34, according to another embodiment of the invention, includes an optic 36 having a posterior surface 38 and an anterior surface 40 with a plurality of diffractive structures 42 in the form of annular diffractive zones superimposed on a base profile 44 of the surface, which are surrounded by a peripheral portion 45 that is devoid of diffractives structures, so as to provide a far focus and a near focus for light transmitted through the lens. Similar to the previous embodiment, the base profile 44 is aspherical with a selected degree of deviation from a putative spherical profile 46 that coincides with the aspherical base profile at small radial distances from the intersection of an optical axis 48 of the lens and the anterior surface 40, as shown schematically in FIGURE 2B. The Cartesian coordinate system depicted in FIGURE 2B allows demonstrating the location of a point on the anterior surface by denoting its radial distance from the intersection of the optical axis and the anterior surface (i.e., the r coordinate) and its sag (z) relative to a plane tangent to the profile at its vertex (i.e., its intersection with the optical axis) and perpendicular to the optical axis.

Each annular diffractive zone is separated from an adjacent zone by a step (e.g., step 50 separating the second zone from the third zone) whose height decreases as the zone's distance from the optical axis increases, thereby providing a gradual shift in division of transmitted optical energy between the near and the far focus of the lens. This reduction in the step heights advantageously ameliorates the unwanted effects of glare perceived as a halo or rings around a distant, discrete light source. The steps are positioned at the radial boundaries of the zones. In this exemplary embodiment, the radial location of a zone boundary can be determined in accordance with the following relation:

$$r_i^2 = (2i+1)\lambda f \qquad\qquad \text{Equation (4)},$$

wherein
$i$ denotes the zone number ($i = 0$ denotes the central zone)
$\lambda$ denotes the design wavelength, and
$f$ denotes a focal length of the near focus.
In some embodiments, the design wavelength $\lambda$ is chosen to be 550 nm green light at the center of the visual response.

The step height between adjacent zones, or the vertical height of each diffractive element at a zone boundary, can be defined according to the following relation:

$$\text{Step height} = \frac{\lambda}{2\,(n_2 - n_1)}\, f_{apodize} \qquad\qquad \text{Equation (5)},$$

wherein
$\lambda$ denotes the design wavelength (e.g., 550 nm),
$n_2$ denotes the refractive index of the material from which the lens is formed,
$n_1$ denotes the refractive index of a medium in which the lens is placed,
and $f_{apodize}$ represents a scaling function whose value decreases as a function of increasing radial distance from the intersection of the optical axis with the anterior surface of the lens.

For example, the scaling function can be defined by the following relation:

$$f_{apodize} = 1 - \left\{ \frac{(r_i - r_{in})}{(r_{out} - r_{in})} \right\}^{exp}, r_{in} \leq r_i \leq r_{out} \qquad\qquad \text{Equation (6)},$$

wherein
$r_i$ denotes the radial distance of the i$^{th}$ zone,
$r_{in}$ denotes the inner boundary of the apodization zone as depicted schematically in FIGURE 2A,
$r_{out}$ denotes the outer boundary of the apodization zone as depicted schematically in FIGURE 2A, and
$exp$ is a value chosen based on the relative location of the apodization zone and a desired reduction in diffractive element step height.

The exponent $exp$ can be selected based on a desired degree of change in diffraction efficiency across the lens surface. For example, $exp$ can take values in a range of about 2 to about 6.

As another example, the scaling function can be defined by the following relation:

$$f_{apodize} = 1 - (\frac{r_i}{r_{out}})^3 \qquad\qquad \text{Equation (7),}$$

wherein

$r_1$ denotes the radial distance of the $i^{th}$ zone, and

$r_{out}$ denotes the radius of the apodization zone.

Referring again to FIGURE 2A, in this exemplary embodiment, each step at a zone boundary is centered about the base profile 44 with half of its height above the base profile and the other half below the profile. Although in this exemplary embodiment, the step heights exhibit a gradual continuous decrease as a function of increasing distance from the optical axis, in other embodiments, a subset of the zones can exhibit the same step heights at their respective boundaries, where these step heights can be different than those at other zone boundaries. Further details regarding selection of the step heights can be found in U.S. Patent No. 5,699,142.

Similar to the previous embodiment, the asphericity of the base profile 44 of the anterior surface 40 of the IOL 34 can be defined in accordance with the above Equation (3). Values similar to those described above can be employed for the conic constant and the higher order deformation coefficients. In particular, selecting a non-zero conic constant (cc) and a sixth order deformation coefficient (*ae*) can be especially beneficial for enhancing image contrast for corneas that are more spherical than normal.

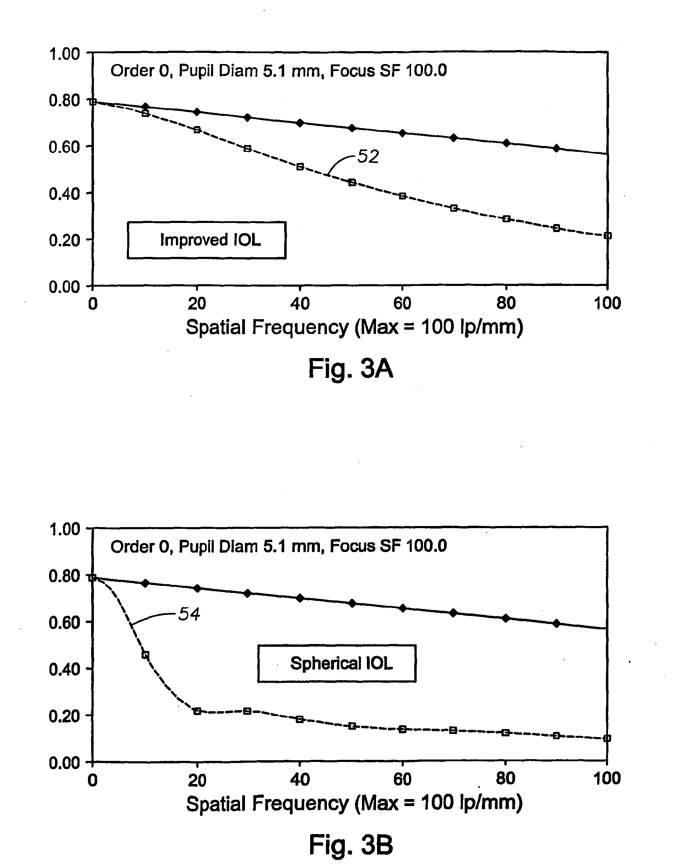

To demonstrate the efficacy of an aspherical diffractive intraocular lens according to the teachings of the invention, FIGURE 3A presents a graph 52 depicting an in-focus modulation transfer function (MTF) calculated in a model eye for an aspherical lens similar to the IOL depicted above in FIGURE 2A, having an optical power of 21 D and an aspherical anterior surface characterized by a conic constant (cc) of -5 and a sixth order deformation constant (ae) of -0.000005 at a wavelength of 550 nm and a pupil diameter of 4.5 mm (5.1 mm at entrance to the eye), while FIGURE 3B presents a graph 54 depicting a corresponding calculated MTF for a substantially identical lens that has a spherical, rather than an aspherical, profile. A comparison of the two graphs 52 and 54 shows that the asphericity of the anterior surface provides considerable improvement in MTF, and consequently in image contrast, even at a high spatial frequency of 100 line pairs per millimeters corresponding to 20/20 vision.

In another set of calculations, modulation transfer functions (MTFs) at spatial frequencies of 50 lp/mm, corresponding to 20/40 vision, as well as at 100 lp/mm, corresponding to 20/20 vision, were calculated for the following five theoretically modeled apodized diffractive intraocular lenses for different corneal shape factors across a range of lens power values. The optical power (at near focus) D, the radius of curvature ($r_1$) of a spherical posterior surface, the radius of curvature ($r_1$) of the anterior surface at its apex, the central thickness ($C_t$) of the lens as well as values of the conic constant (cc) and the sixth order deformation constant (ae) for these theoretically modeled lenses are presented in the table below:

**TABLE 1**

| Lens | Power (D) | r1 (mm) | Ct (mm) | r2 (mm) | cc | ae |
|------|-----------|---------|---------|---------|------|-----------|
| A | 16.5 | 16.5 | 0.553 | -66.297 | -8 | -0.00001 |
| B | 19.5 | 16.5 | 0.615 | -34.587 | -10 | -0.00001 |
| C | 23.5 | 13.5 | 0.7 | -29.523 | -5 | -0.000005 |
| D | 27.5 | 11 | 0.787 | -28.186 | -3.5 | -0.000003 |
| E | 30 | 9 | 0.845 | -37.411 | -1.8 | -0.000005 |

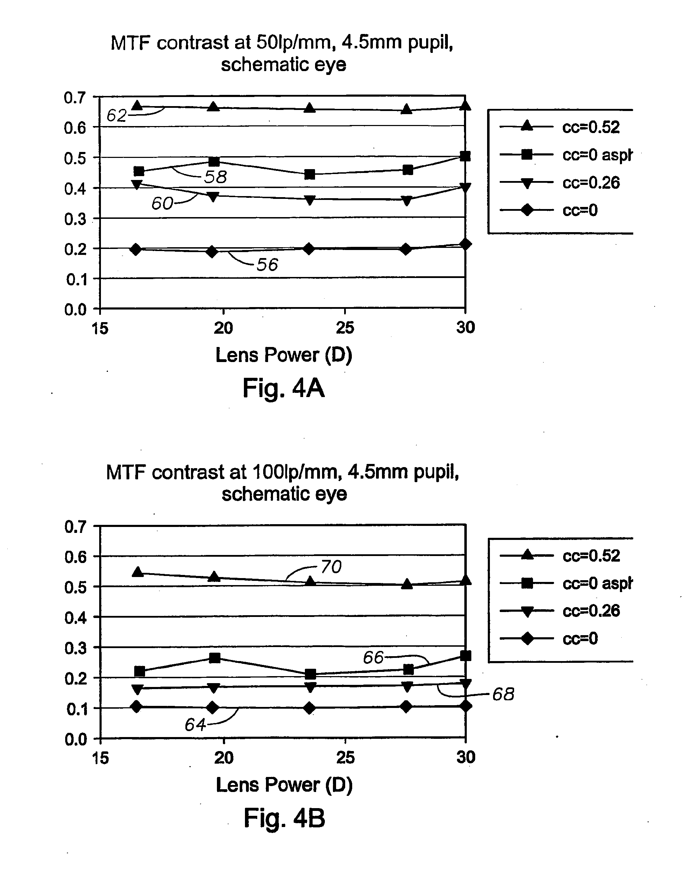

FIGURE 4A presents a plurality of graphs depicting modulation transfer functions calculated at 50 lp/mm and a pupil size of 4.5 mm for each of the apodized diffractive aspherical intraocular lenses listed in the above Table 1, combined with corneas exhibiting a range of asphericity - from a spherical cornea with a corneal conic constant of zero to one having a severe flattening with a corneal conic constant of -0.52 (minus .52) -, as well as a control graph exhibiting corresponding MTFs for substantially identical lenses but with spherical, rather than aspherical, surfaces combined with a spherical cornea. More particularly, a graph 56 depicts MTF values obtained for such control spherical lenses in combination with a spherical cornea while another graph 58 depicts MTF values obtained for each of the aspherical lenses A-E with a spherical cornea. A comparison of graph 56 with graph 58 shows that aspherical lenses A-E provide a much enhanced image contrast (the MTF values corresponding to the aspherical lenses are at least a factor of 2 greater than those for corresponding spherical lenses) relative to the substantially identical spherical lenses. Graphs 60 and 62 present MTF values for each of the lenses A-E in combination, respectively, with a cornea exhibiting an asphericity characterized by a conic constant of about -0.26 (minus .26) - a level of asphericity often reported for an average eye - and a cornea exhibiting an asphericity characterized by a conic constant of about -.52 (minus .52) - a level of corneal flattening that minimizes spherical aberration. This illustrative data indicates that the aspherical lenses can provide good image contrast for a wide range of corneal shapes.

Additional theoretical MTF values calculated at a higher spatial frequency of 100 lp/mm and at a wavelength of 550 nm and a pupil size of 4.5 mm are presented in FIGURE 4B. A comparison of graph 64, presenting MTF values corresponding to lenses substantially identical to the above lenses A-E but with spherical profiles combined with spherical corneas, with graph 66, presenting MTF values corresponding to the aspherical lenses A-E combined with spherical corneas, indicates that the aspherical lenses provide much greater image contrast even at a much higher spatial frequency of 100 lp/mm, corresponding to 20/20 vision. Similar data for lenses A-E in combination with a cornea exhibiting an asphericity characterized by a conic constant of- 0.26 (minus 0.26) and a cornea characterized by a conic constant of - 0.52 (minus .52) are also provided (graphs 68 and 70) to illustrate that the aspherical lenses A-E provide image contrast enhancement over a range of corneal conditions even at high spatial frequencies.

In the above exemplary data, calculated modulated transfer functions (MTF) for apodized diffractive lenses were presented. The MTFs were calculated by utilizing a ray tracing procedure in which variation of diffraction efficiency across the diffractive zones is incorporated in a manner described in more detail below. In general, MTF calculations for an apodized diffractive lens, e.g., one having diffractives step heights that vary across the surface, are more complex than corresponding calculations for a diffractive lens having uniform step sizes across its entire surface. In the latter case, the MTF can be calculated in a conventional manner and then rescaled by assuming that the light that is not directed to the focus of interest acts to reduce the image contrast. The MTF contrast values can be multiplied by a diffraction efficiency, except for the point at zero spatial frequency that is set to unity. This is equivalent to assuming that the image plane is evenly illuminated by the light energy that is not focused, with all spatial frequencies of the defocused light equally represented. Although in practice the defocused light has spatial structure in the image plane, it is highly defocused and hence does not significantly affect the overall form of the MTF. In the former case, as noted above, principles of physical optics should be employed to calculate optical properties of an apodized diffractive lens. One method of the invention for calculating optical properties of an apodized diffractive lens models apodization as different levels of reduction in optical transmission through different regions of the lens.

By way of example, in one exemplary method according to the invention for calculating an MTF for an apodized diffractive lens having progressively decreasing step heights (e.g., the lens schematically shown in the above FIGURE 2A), the step heights are modeled to correspond to local diffraction efficiencies by assuming that the respective lens surface is a diffraction grating with an appropriate optical path length at each diffractive step. For example, to calculate local diffraction efficiencies for a lens having an aspherical anterior surface whose diffractive step heights are characterized by the above Equations (5) and (7), the diffraction efficiency (DE) for directing light into diffraction order p at the design wavelength ($\lambda$) is given by the following Equations (8) and (9) in which $\alpha$ is a fraction of $2\pi$ phase delay introduced at a step having a step height ($h$), and $n_1$ and $n_2$ are the indices of refraction of the lens material and the surrounding medium, respectively:

$$\alpha = h * (n_2 - n_1) / \lambda \qquad \text{Equation (8)},$$

$$DE_p = \text{sinc}^2(\alpha - p) \qquad \text{Equation (9)},$$

wherein $\text{sinc}(x) = \dfrac{\sin(\pi x)}{(\pi x)}$. Hence, the diffraction efficiency can be determined at any point on the surface by utilizing the local step height provided in Equations (5) and (7). In this manner, the diffraction efficiency provides the local fraction of incident light energy that is directed towards an image of a particular order, thereby providing the effective apodization transmission function.

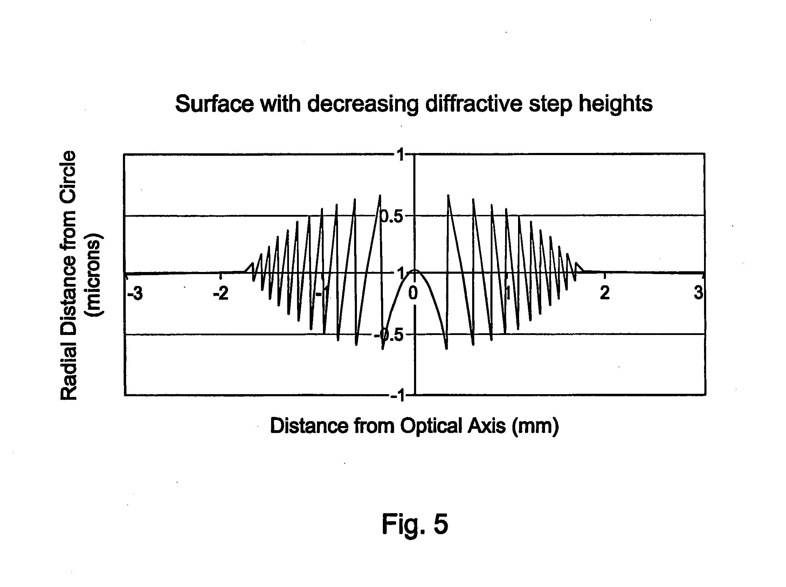

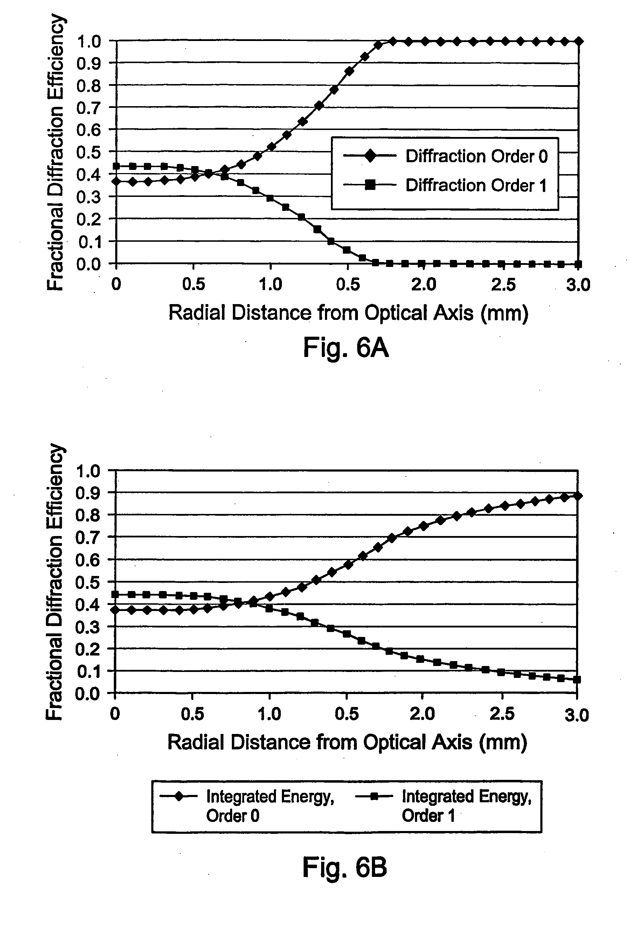

By way of example, the diffraction efficiency of an exemplary apodized diffractive lens having progressively decreasing step heights shown schematically in FIGURE 5 (the base line is subtracted for clarity) was calculated by employing the above approach. FIGURE 6A depicts graphs corresponding to the calculated fractional diffraction efficiency for the zeroth order and the first order, which correspond to far and near focus respectively, as a function of radial distance from the lens' optical axis. As noted above, the local diffraction efficiency defines an apodization function of the lens. However, the total energy that is directed to a focus is required to rescale an MTF appropriately. To this end, the diffraction efficiency can be integrated over a selected aperture, e.g., pupil area, to provide the total energy directed to each focus. By way of example, FIGURE 6B presents graphs corresponding to total energy directed to the zeroth and the first order foci of the exemplary lens as a function of pupil radius, obtained by integration of the diffraction efficiencies depicted in FIGURE 6A.

The above method for calculating an MTF of an apodized diffractive lens can be incorporated into a commercial raytrace program, such as OSLO premium ray-tracing program marketed by Lambda Research Corporation of Littelton, Massachusetts, U.S.A., to rescale the points of a conventionally-calculated MTF by a fraction of energy that is directed to a focus of interest (apart from the point at zero spatial frequency that is set to unity) to account for the energy directed to the other orders.

In some embodiments, the surface having the diffractive structures can have a spherical base curve, and the other surface (i.e., the surface lacking the diffractive structures) can have a degree of asphericity selected based on the teachings of the invention, such as those described above.

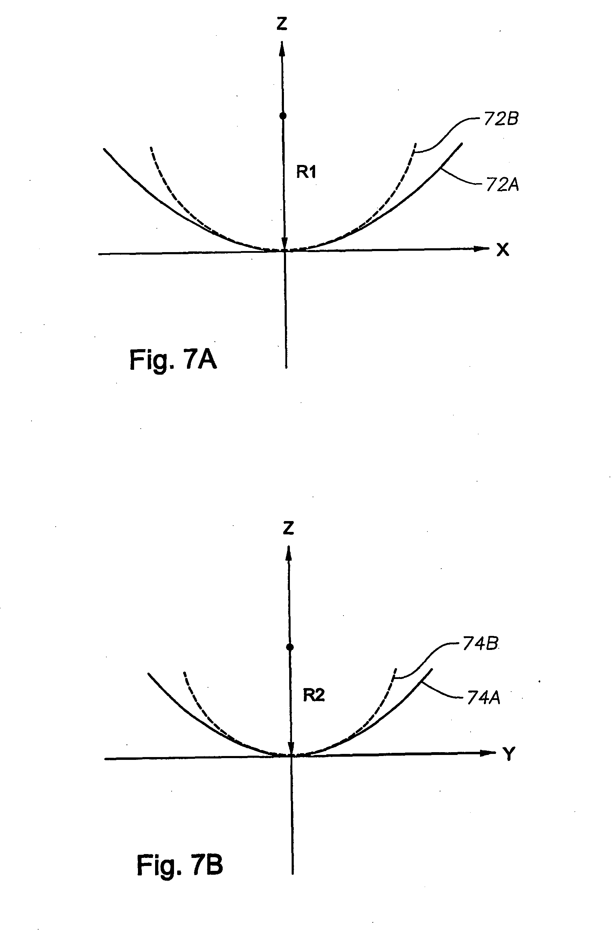

In another embodiment, an apodized diffractive intraocular lens (IOL) of the invention can have one or two toric surfaces that exhibit two different optical powers along two orthogonal surface directions. Such toric IOLs can be employed, for example, to correct astigmatism. At least one of the toric surfaces can exhibit an asphericity along one or both of the two orthogonal directions. For example, with reference to FIGURE 7A, the toric surface in one of the two directions (herein identified with the x coordinate) can be characterized by an aspherical profile 72A having a central curvature $R_1$ at its vertex (i.e., intersection of an optical axis of the lens with the surface) and a selected deviation from a putative spherical profile 74B that substantially coincides with the aspherical profile at small radial distances. As shown in FIGURE 7B, along the other direction (herein identified with the y coordinate), a profile 74A of the toric surface can be characterized by a central curvature $R_2$, that is different than $R_1$, and a selected deviation from a putative spherical profile 72B that substantially coincides with the aspherical profile at small radial distances. The toric surface having asphericity along one or both of its orthogonal surface directions can also include uniform or non-uniform diffractive structures within an apodization zone, such as the structures depicted in the previous embodiment. Alternatively, the toric surface exhibiting asphericity can be the lens surface that is devoid of diffractive structures. In some embodiments, both lens surfaces of a toric lens (i.e., the one having diffractive structures and the one lacking such structures) can exhibit a selected degree of asphericity in one or both orthogonal surface directions.

Although the above embodiments are directed to intraocular lenses, it should be understood that the teachings of the invention, including the use of aspherical surface profiles to enhance image contrast, can be applied to other ophthalmic apodized diffractive lenses, e.g., contact lenses.

Those having ordinary skill in the art will appreciate that various modifications can be made to the above embodiments without departing from the scope of the invention.

**Claims**

1. An apodized diffractive lens (34), comprising:

   an optic (36) having an anterior surface (40) and a posterior surface (38) each having a base profile (44), said anterior surface having a plurality of annular or concentric diffractive structures (42) superimposed on said base profile about an optical axis (48) within an apodization zone thereof,
   **characterized in that** at least of one said anterior or posterior surfaces has a toric shape with two different optical power values along two orthogonal directions along the surface so as to generate a far focus and near focus and exhibits an aspherical base profile (72A) along at least one of said surface directions.

2. The lens of claim 1, wherein said apodization zone of the lens surface is surrounded by a portion of the lens surface (45) substantially devoid of diffractive structures (42).

3. The lens of claim 1 or claim 2, wherein said diffractive structures (42) are separated from one another by a plurality of steps (50) having substantially uniform heights.

4. The lens of claim 1 or claim 2, wherein said diffractive structures (42) are separated from a neighboring structure by a step (50) having a height that progressively decreases as a function of distance from a central axis (48) of said optic.

5. The lens of any one of claims 1 to 4, wherein said aspherical base profile (72A) is **characterized by** the following relation:

$$z = \frac{cR^2}{1 + \sqrt{1 - (1 + cc)c^2 R^2}} + adR^4 + aeR^6,$$

wherein

$z$ denotes a sag of the surface parallel to an axis (z), e.g., the optical axis, perpendicular to the surface,
$c$ denotes a curvature at the vertex of the surface,
$cc$ denotes a conic coefficient,
$R$ denotes a radial position on the surface,
$ad$ denotes a fourth order deformation coefficient, and
$ae$ denotes a sixth order deformation coefficient.

6. The lens of any one of claims 1 to 5, wherein said lens comprises an intraocular lens (IOL).

7. The lens of claim 6, wherein an optical system comprising said IOL and a patient's eye in which said IOL is implanted exhibits a modulation transfer function (MTF) greater than about 0.2 as calculated in a model eye at a spatial frequency of about 50 lp/mm, a wavelength of about 550 nm and a pupil size of about 4.5 mm.

8. The lens of claim 6, wherein an optical system comprising said lens and a patient's eye in which said lens is implanted exhibits a modulation transfer function (MTF) greater than about 0.1 as calculated in a model eye at a spatial frequency of about 100 lp/mm, a wavelength of about 550 nm and a pupil size of about 4.5 mm.

9. The IOL of claim 6, wherein an optical system comprising said lens and a patient's eye in which the lens is implanted exhibit a modulation transfer function (MTF) greater than about 0.2 as calculated in a model eye at a spatial frequency of about 100 lp/mm and a wavelength of about 550 nm for a pupil diameter of about 4 mm.

10. The IOL of claim 9, wherein said MTF is greater than 0.3.

11. The IOL of claim 9, wherein said MTF is greater than 0.4.

12. The IOL of claim 9, wherein said MTF is in a range of 0.2 to 0.5.

13. The lens of any one of claims 1 to 5, wherein said lens comprises a contact lens.

14. The lens of any one of claims 1 to 13, wherein said optic is formed of any of acrylic, silicone, or hydrogel polymeric material.

**Patentansprüche**

1. Apodisierte Beugungslinse (34), mit:

einer Optik (36) mit einer vorderen Oberfläche (40) und einer hinteren Oberfläche (38) mit jeweils einem Basisprofil (44),
wobei die vordere Oberfläche eine Mehrzahl ringförmiger oder konzentrischer Beugungsstrukturen (42) hat, die das Basisprofil um eine optische Achse (48) in einer Apodisierungszone desselben überlagern,
**dadurch gekennzeichnet, dass** mindestens eine der vorderen oder hinteren Oberfläche eine Torusform mit zwei verschiedenen optischen Wirkungswerten entlang zweier senkrecht zueinander stehenden Richtungen entlang der Oberfläche hat, um einen Fernfokus und einen Nahfokus zu erzeugen, und ein asphärisches Basisprofil (72a) entlang mindestens einer der Oberflächenrichtungen aufweist.

2. Linse nach Anspruch 1, bei der die Apodisierungszone der Linsenoberfläche von einem Abschnitt der Linsenoberfläche (45) umgeben ist, der im Wesentlichen frei ist von Beugungsstrukturen (42).

3. Linse nach Anspruch 1 oder 2, wobei die Beugungsstrukturen (42) durch eine Mehrzahl Stufen (50) mit im Wesentlichen gleicher Höhe voneinander getrennt sind.

4. Linse nach Anspruch 1 oder 2, wobei die Beugungsstrukturen (42) von einer benachbarten Struktur durch eine Stufe (50) mit einer Höhe, die als Funktion des Abstands von der Mittelachse (48) der Optik progressiv abnimmt, getrennt sind.

5. Linse nach einem der Ansprüche 1 bis 4, wobei das asphärische Basisprofil (72a) durch die folgende Beziehung **gekennzeichnet** ist:

$$z = \frac{cR^2}{1+\sqrt{1-(1+cc)c^2 R^2}} + adR^4 + aeR^6;$$

dabei bedeuten
z ein Durchhängen der Oberfläche parallel zu einer Achse (z), z. B. der optischen Achse, senkrecht zur Oberfläche,
c die Krümmung im Scheitel der Oberfläche,
cc einen konischen Koeffizienten,
R eine radiale Position auf der Oberfläche,
ad einen Deformationskoeffizienten vierter Ordnung, und
ae einen Deformationskoeffizienten sechster Ordnung.

6. Linse nach einem der Ansprüche 1 bis 5, wobei die Linse eine Intraokularlinse (IOL) aufweist.

7. Linse nach Anspruch 6, wobei ein die IOL aufweisendes optisches System und ein Auge des Patienten, in das die IOL implantiert wird, eine Modulationstransferfunktion (MTF) aufweist, die größer ist als ca. 0,2 gemäß einer Berechnung mit einem Modellauge bei einer Raumfrequenz von ca. 50 lp/mm, einer Wellenlänge von ca. 550 nm und einer Pupillengröße von ca. 4,5 mm.

8. Linse nach Anspruch 6, wobei ein die Linse aufweisendes optische System und das Auge des Patienten, in das die Linse implantiert wird, eine Modulationstransferfunktion (MTF) aufweist, die größer ist als ca. 0,1 gemäß einer Berechnung mit einem Modellauge bei einer Raumfrequenz von ca. 100 lp/mm, einer Wellenlänge von ca. 550 nm und einer Pupillengröße von ca. 4,5 mm.

9. IOL nach Anspruch 6, wobei ein die Linse aufweisendes optische System und das Auge des Patienten, in das die Linse implantiert wird, eine Modulationstransferfunktion (MTF) aufweist, die größer ist als ca. 0,2 gemäß einer Berechnung mit einem Modellauge bei einer Raumfrequenz von ca. 100 lp/mm, einer Wellenlänge von ca. 550 nm und einer Pupillengröße von ca. 4 mm.

10. IOL nach Anspruch 9, wobei die MTF größer ist als 0,3.

11. IOL nach Anspruch 9, wobei die MTF größer ist als 0,4.

12. IOL nach Anspruch 9, wobei die MTF in einem Bereich von 0,2 bis 0,5 liegt.

13. Linse nach einem der Ansprüche 1 bis 5, wobei die Linse eine Kontaktlinse aufweist.

14. Linse nach einem der Ansprüche 1 bis 13, wobei die Optik aus einem der Materialien Acryl, Silikon oder Hydrogelpolymer besteht.

## Revendications

1. Lentille diffractive apodisée (34) comprenant :

   un élément optique (36) ayant une surface antérieure (40) et une surface postérieure (38) ayant chacune un profil de base (44),
   ladite surface antérieure ayant une pluralité de structures diffractives annulaires ou concentriques (42) superposées sur ledit profil de base autour d'un axe optique (48) dans une zone d'apodisation de celles-ci,
   **caractérisée en ce qu'**au moins une desdites surfaces antérieures et postérieures a une forme torique avec deux valeurs de puissance optique différentes le long de deux directions orthogonales le long de la surface afin de générer un foyer loin et un foyer proche et présente une profil de base asphérique (72A) le long d'au moins une desdites directions de surface.

**2.** Lentille selon la revendication 1, dans laquelle ladite zone d'apodisation de la surface de lentille est entourée par une partie de la surface de lentille (45) essentiellement dépourvue de structures diffractives (42).

**3.** Lentille selon la revendication 1 ou 2, dans laquelle lesdites structures diffractives (42) sont séparées les unes des autres par une pluralité de paliers (50) ayant des hauteurs essentiellement uniformes.

**4.** Lentille selon la revendication 1 ou 2, dans laquelle lesdites structures diffractives (42) sont séparées d'une structure voisine par un palier (50) ayant une hauteur qui diminue progressivement en fonction de la distance à partir d'un axe central (48) de ladite optique.

**5.** Lentille selon l'une quelconque des revendications 1 à 4, dans laquelle ledit profil de base asphérique (72A) est **caractérisé par** la relation suivante :

$$ z = \frac{cR^2}{1 + \sqrt{1 - (1 + cc)c^2 R^2}} + adR^4 + aeR^6, $$

dans laquelle
$z$ représente un gauchissement de la surface parallèlement à un axe (z), par exemple l'axe optique, de manière perpendiculaire à la surface,
$c$ représente une courbure au niveau du sommet de la surface,
$cc$ représente un coefficient conique,
$R$ représente une position radiale sur la surface,
$ad$ représente un coefficient de déformation de quatrième ordre, et
$ae$ représente un coefficient de déformation de sixième ordre.

**6.** Lentille selon l'une quelconque des revendications 1 à 5, dans laquelle ladite lentille comprend une lentille intraoculaire (IOL).

**7.** Lentille selon la revendication 6, dans laquelle un système optique comprenant ladite lentille intraoculaire et un oeil de patient dans lequel ladite lentille intraoculaire est implantée présente une fonction de transfert de modulation (MTF) supérieure à environ 0,2, le calcul étant effectué dans un modèle d'oeil à une fréquence spatiale d'environ 50 lp/mm, une longueur d'onde d'environ 550 nm et une taille de pupille d'environ 4,5 mm.

**8.** Lentille selon la revendication 6, dans laquelle un système optique comprenant ladite lentille et un oeil de patient dans lequel ladite lentille est implantée présente une fonction de transfert de modulation (MTF) supérieure à environ 0,1 le calculé tant effectué dans un modèle d'oeil à une fréquence spatiale d'environ 100 lp/mm, une longueur d'onde d'environ 550 nm et une taille de pupille d'environ 4,5 mm.

**9.** Lentille intraoculaire selon la revendication 6, dans laquelle un système optique comprenant ladite lentille et un oeil de patient dans lequel la lentille est implantée présente une fonction de transfert de modulation (MTF) supérieure à environ 0,2, le calculé étant effectué dans un modèle d'oeil à une fréquence spatiale d'environ 100 lp/mm, une longueur d'onde d'environ 550 nm et un diamètre de pupille d'environ 4 mm.

**10.** Lentille intraoculaire selon la revendication 9, dans laquelle ladite MTF est supérieure à 0,3.

**11.** Lentille intraoculaire selon la revendication 9, dans laquelle ladite MTF est supérieure à 0,4.

**12.** Lentille intraoculaire selon la revendication 9, dans laquelle ladite MTF est dans la plage de 0,2 à 0,5.

**13.** Lentille selon l'une quelconque des revendications 1 à 5, dans laquelle ladite lentille comprend une lentille de contact.

**14.** Lentille selon l'une quelconque des revendications 1 à 13, dans laquelle ladite optique est constituée d'un quelconque parmi les matériaux polymères acrylique, silicone ou hydrogel.

**Fig. 1A**

**Fig. 1B**

**Fig. 1C**

Fig. 2A

Fig. 2B

Fig. 3A

Fig. 3B

MTF contrast at 50lp/mm, 4.5mm pupil,
schematic eye

Fig. 4A

MTF contrast at 100lp/mm, 4.5mm pupil,
schematic eye

Fig. 4B

Surface with decreasing diffractive step heights

Radial Distance from Circle (microns)

Distance from Optical Axis (mm)

Fig. 5

Fig. 6A

Fig. 6B

Fig. 7A

Fig. 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004156014 A1, Piers
- US 5824074 A, Koch
- US 5699142 A